(19) Europäisches Patentamt
European Patent Office
Office européen des brevets

(11) Publication number: **0 023 083**
**A1**

(12) **EUROPEAN PATENT APPLICATION**

(21) Application number: 80302012.2

(22) Date of filing: 16.06.80

(51) Int. Cl.³: C 07 D 233/78
C 07 D 233/86, C 07 D 233/32
A 61 K 31/415

(30) Priority: 27.06.79 GB 7922392

(43) Date of publication of application:
28.01.81 Bulletin 81/4

(84) Designated Contracting States:
AT BE CH DE FR GB IT LI NL SE

(71) Applicant: BEECHAM GROUP LIMITED
Beecham House Great West Road
Brentford, Middlesex(GB)

(72) Inventor: Cassidy, Frederick
12 Peterswood
Harlow Essex(GB)

(72) Inventor: Moore, Richard William Moore
34 Sunningdale Bishop
Stortford Hertfordshire(GB)

(74) Representative: Dawson, Hugh Bainforde, Dr. et al,
European Patent Attorney BEECHAM
PHARMACEUTICALS Yew Tree Bottom Road
Epsom, Surrey KT18 5XQ.(GB)

(54) **Oxodiazole derivatives, processes for their production and pharmaceutical composition containing them.**

(57) Compounds of the formula (I):

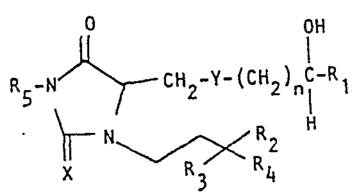

wherein:

X is O, S or $H_2$;
Y is $-CH_2-CH_2-$, $-CH=CH-$ or $-C\equiv C-$;
n is 1 to 5;
$R_1$ is $C_{1-4}$ alkyl;
$R_2$ is hydrogen or $C_{1-4}$ alkyl;
$R_3$ is a hydroxy or protected hydroxy;
$R_4$ is $C_{1-9}$ alkyl, $C_{3-8}$ cycloalkyl or $C_{3-8}$ cycloalkyl-$C_{1-6}$ alkyl; and
$R_5$ is hydrogen or $C_{1-6}$ alkyl; and salts thereof having useful pharmacological activity, compositions containing them and processes for their preparation.

EP 0 023 083 A1

## Active Compounds

This invention relates to novel compounds having pharmacological activity, to a process for their preparation, to intermediates useful in that process and to pharmaceutical compositions containing them.

German Offenlegungsschrift No. 2724948 discloses that compounds of the general formula (A);

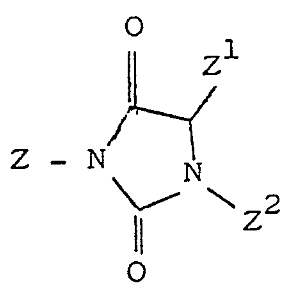

(A)

wherein Z is hydrogen or alkyl; one of $Z^1$ and $Z^2$ is a group - $CH_2-X-X^1-X^2$ in which X is phenylene, -C≡C-, *cis*- or trans- -CH=CH- or $-CH_2-CQ_2-$, where each radical Q independently of the other is hydrogen and/or alkyl or the two radicals Q together are $C_{4-6}$ alkylene, $X^1$ is a covalent bond or a straight or branched $C_{1-6}$ alkylene chain, in which one methylene group is optionally substituted by an oxa (-O-) group, with the proviso that at least one carbon atom separates the oxa group from a -C≡C-, -CH=CH- or CO group, and $X^2$ is tetrazolyl, carboxyl, carboxamide, hydroxymethylene and/or alkoxycarbonyl; and the other one of $Z^1$ and $Z^2$

is a group $-Y-Y^1-Y^2-Y^3$ in which Y is $-CR_2-CH_2-$, where each radical R independently of the other is hydrogen and/or methyl, $Y^1$ is carbonyl, methylene, methylene substituted by a hydroxy group or methylene substituted by a hydroxy and alkyl group, $Y^2$ is a covalent bond or straight-chain or branched $C_{1-7}$ alkylene optionally substituted on the carbon atom adjacent to $Y^1$ by one or two mutually independent alkyl, bicycloalkyl or cycloalkyl groups, $Y^3$ is hydrogen, hydroxy, $C_{1-7}$ (preferably $C_{1-4}$) alkoxy, cycloalkyl, bicycloalkyl, phenyl, benzyl, phenoxy or benzyloxy, where each phenyl, benzyl, phenoxy or benzyloxy group may be substituted in the benzene ring by one or more hydroxy, halogen, nitro, amino, acylamino, alkenyl, alkoxy, phenyl and/or alkyl groups, which themselves may be substituted by one or more halogens or Y is a bond, $-CH_2-$ or $-CH_2.CH_2-$ and $Y^1$, $Y^2$ and $Y^3$ together are cycloalkyl which is substituted by a hydroxy group which is preferably separated by 3 carbon atoms from the hydrantoin ring, have similar pharmacological activity to natural prostaglandins.

We have now discovered a class of compounds which have useful pharmocological activity and which are structurally distinct from the compounds disclosed in Offenlegungsschrift No. 2724948.

Accordingly the present invention provides a compound of formula (I):

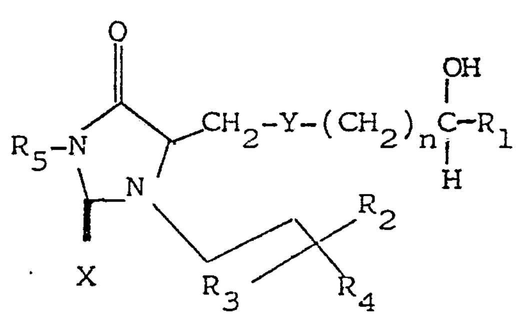

(I)

wherein:

X is O, S or $H_2$;

Y is $-CH_2-CH_2-$, $-CH=CH-$ or $-C\equiv C-$;

n is 1 to 5;

$R_1$ is $C_{1-4}$ alkyl;

$R_2$ is hydrogen or $C_{1-4}$ alkyl;

$R_3$ is a hydroxy or protected hydroxy;

$R_4$ is $C_{1-9}$ alkyl, $C_{3-8}$ cycloalkyl or $C_{3-8}$ cycloalkyl-$C_{1-6}$ alkyl; and

$R_5$ is hydrogen or $C_{1-6}$alkyl; and salts thereof.

Particularly suitable compounds within formula (I) include those where X is O.

Preferably Y is $-CH_2-CH_2-$ or *cis*- $-CH=CH-$.

Preferably n is 3, 4 or 5, in particular 4.

Suitable examples of $R_1$ include methyl and ethyl.

Suitable examples of $R_2$ include hydrogen, methyl and ethyl. More suitably $R_2$ is hydrogen or methyl, preferably methyl.

Suitable protected hydroxyl groups $R_3$ include readily hydrolysable groups such as acylated hydroxy groups in which the acyl moiety contains 1 to 4 carbon atoms, for example the acetoxy group; and hydroxy groups etherified by readily removable inert groups such as the benzyl group or like groups. Preferably $R_3$ is hydroxyl.

Suitable groups $R_4$ when $R_4$ is an alkyl group include $C_{4-9}$ alkyl groups. Such $C_{4-9}$ alkyl groups may be straight chain alkyl groups, such as n-butyl, n-pentyl, *n*-hexyl and *n*-heptyl, or may be alkyl groups branched by one or two methyl groups ( at the same or different carbon atoms ). Thus for example, $R_4$ may be a group $CH_2R_7$, $CH(CH_3)R_7$ or $C(CH_3)_2R_7$, wherein $R_7$ is a straight chain alkyl group such that the carbon content of the resultant group $R_4$ is 4 to 9.

In general preferred groups $R_4$ when $R_4$ is an

alkyl group include straight chain pentyl, hexyl and heptyl groups. Of these, straight chain hexyl is often the most useful. Other preferred groups $R_4$ include groups $CH(CH_3)R_7$ and $C(CH_3)_2R_7$ wherein $R_7$ is straight chain butyl, pentyl and hexyl.

Other suitable examples of $R_4$ when $R_4$ is an alkyl group include the lower alkyl groups, that is when $R_4$ is a $C_{1-4}$ alkyl group.

When $R_4$ is or contains a $C_{3-8}$ cycloalkyl moiety, the moiety may be cyclopropyl. The moiety may also preferably be a $C_{5-8}$ cycloalkyl moiety in particular a cyclohexyl moiety. Examples of suitable $C_{1-6}$ alkyl moieties when $R_4$ is a $C_{3-8}$ cycloalkyl-$C_{1-6}$ alkyl group include methyl, ethyl, propyl, butyl and pentyl.

Suitable examples of $R_5$ include hydrogen, methyl, ethyl, <u>n</u>- and <u>iso</u>-propyl, <u>n</u>-, <u>sec</u>- and <u>tert</u>-butyl.

Some of the compounds of formula (I) may form conventional salts. Such salts include those with alkali and alkaline earth metals, including suitably sodium and potassium, and acid addition salts when X is $H_2$.

From the aforesaid it will be seen that one particularly suitable group of compounds within formula (I) is of formula (II):

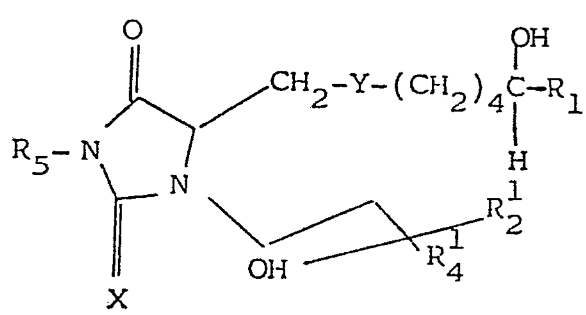

(II)

BAD ORIGINAL

wherein:

X, Y $R_1$ and $R_5$ are as defined in formula (I);

$R_2^1$ is hydrogen, methyl or ethyl; and

$R_4^1$ is $C_{1-9}$ alkyl; and salts thereof.

In formula (II) suitably X is O.

Suitably Y is $-CH_2-CH_2-$ or cis- -CH=CH.

Suitably $R_1$ is methyl.

$R_2^1$ is more suitably hydrogen or methyl, preferably methyl.

While $R_4^1$ may be a $C_{1-9}$ alkyl group, it is normally a $C_{4-9}$ alkyl group. In such cases suitable and preferred straight chain and branched groups $R_4^1$ include those previously described as suitable and preferred for the group $R_4$ and $R_4$ is a $C_{4-9}$ alkyl group. Such preferred groups $R_4^1$ include straight chain pentyl, hexyl, and heptyl and of these normally the most useful is straight chain hexyl. Other preferred groups $R_4^1$ include $CH(CH_3)R_7^1$ and $C(CH_3)_2R_7^1$ wherein $R_7^1$ is straight chain butyl, pentyl or hexyl.

Suitably $R_5$ is hydrogen, methyl or ethyl.

A further group of compounds of interest within formula (I) is of formula (III):

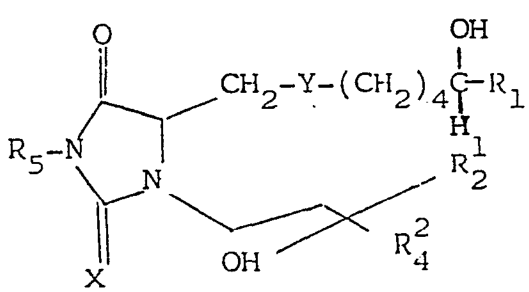

(III)

wherein:

X, Y, $R_1$ and $R_5$ are as defined in formula (I):

$R_2^1$ is hydrogen, methyl or ethyl;

$R_4^2$ is a group of formula (IV):

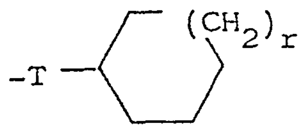

(IV)

wherein T is a bond, or a C alkylene group which may be straight chain or branched by one or two methyl groups at the same or different carbon atoms; and

r is 0 to 3;

and salts thereof.

In formula (III) suitably X is O.

Suitably Y is $-CH_2-CH_2-$ or <u>cis</u>- -CH=CH-.

Suitably $R_1$ is methyl.

$R_2^1$ is more suitably hydrogen or methyl, preferably methyl.

In formula (IV) often T will be a group $-(CH_2)q-$ wherein q is 0 to 4, preferably 0. Also suitably r is 1.

Suitably $R_5$ is hydrogen, methyl or ethyl.

It will of course be realised that the compounds of the formula (I) have asymmetric centres, and thus are capable of existing in a number of stereoisomeric forms. The invention extends to each of these stereoisomeric forms, and to mixtures thereof. The different stereoisomeric forms may be separated one from the other by the usual methods.

The present invention further provides a process for the preparation of the compounds of the formula (I) which process comprises the cyclisation of a compound of formula (V):

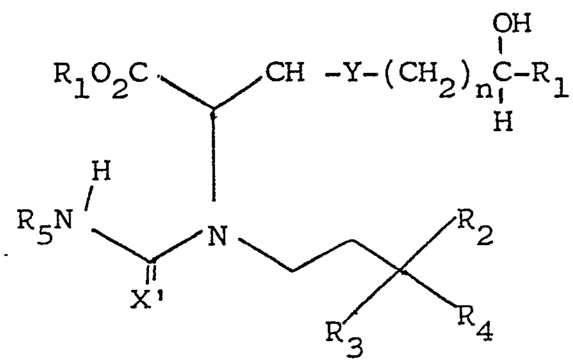

(V)

wherein the variable groups are as hereinbefore defined and X' is O or S; and thereafter if desired or necessary converting X, Y, $R_1$, $R_3$ and $R_5$ in the thus formed compound into other variables X, Y, $R_1$, $R_3$ and $R_5$.

When $R_5$ is alkyl in the compound of formula (V), then the compound of the formula (V) is conveniently prepared *in situ* during the reaction of a compound of the formula (VI):

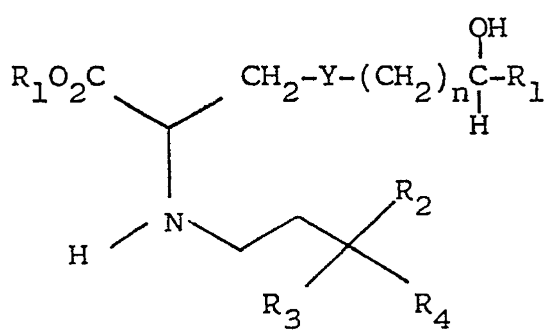

(VI)

with $R_5NCX'$ wherein $R_5$ is alkyl and X' is O or S.

This preferred process is suitably carried out under reflux in an inert solvent such as benzene and the like. It should be stated that when in this reaction $R_5$ is a sterically hindered group then this reaction may proceed only as far as the uncyclised compound of formula (V), in which case the necessary cyclisation of the compound (V) can be achieved with a strong base, such a sodium hydride or sodium ethoxide, in a dry organic solvent. Sodium ethoxide in benzene, or potassium _t_-butoxide in toluene, benzene or hexamthyl phosphoramide are suitable reagents.

When $R_5$ is hydrogen in the compound of formula (V), then the compound of formula (V) is conveniently formed _in situ_ during the reaction of a compound of formula (IV) with a salt $M^+\bar{C}NX^1$ wherein $M^+$ is a metal ion, preferably potassium, in the presence of acid. The acid for this reaction, which yields a compound of formula (I), is suitably provided by using an acid addition salt of the compound of formula (VI), or by carrying out the reaction in aqueous acid.

Intermediates of the formula (VI) may be prepared by reacting a compound of formula (VII):

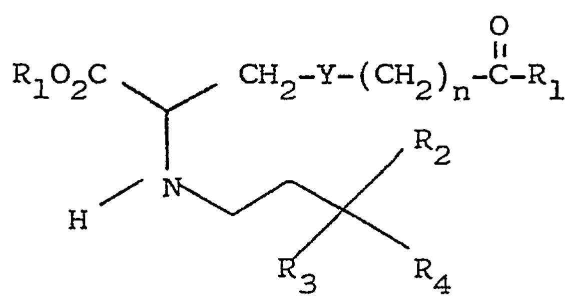

(VII)

wherein the variables are as defined in formula (I), and $R_2$ and $R_3$ may also together form an oxo group, with a carbonyl reducing agent.

The necessary reduction of a compound of the formula (VII) may be carried out with any of the usual carbonyl-reducing agents under conditions which will be apparent to the skilled man.

Suitable examples of carbonyl reducing agents are borohydrides, such as sodium or lithium borohydrides, which may be used in a solvent such as diglyme, a lower alcohol or an ether.

Intermediates of the formula (VII) may be prepared by methods described for the intermediates to compounds forming the subject matter of published European Patent Application No 79301145.3, or by methods analogous thereto.

Alternatively, intermediates of the formula (VII) wherein $R_2$ and $R_3$ together form an oxo group may be prepared by reacting a compound of formula (VIII):

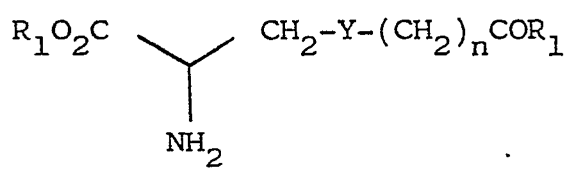

(VIII)

wherein the variables are as hereinbefore defined, with a compound of formula (IX): 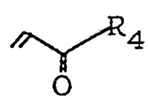

wherein $R_4$ is as hereinbefore defined.

The present invention also provides an alternative process for the preparation of a compound of the formula (I) wherein X is O which process comprises reacting a compound of the formula (X):

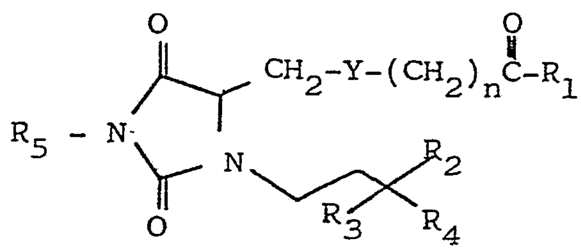

(X)

wherein the variables are as defined in formula (VII) with a carbonyl-reducing agent, and thereafter if desired or necessary converting Y, $R_1$, $R_3$ and $R_5$ in the thus formed compound into other variables Y, $R_1$, $R_3$ and $R_5$.

The reduction of a compound of formula (X) may be carried out as hereinbefore described for the reduction of compounds of the formula (VII).

The compounds of formula (X) are within the subject matter of published European Patent Application No 79301145.3 and their preparation is fully described therein.

The conversion of a compound of the formula (I) to another compound of the formula(I) wherein X, Y, $R_3$ and/or $R_5$ are altered, when desired or necessary, may be achieved in any conventional manner.

For example compounds wherein X is S may be converted to compounds wherein X is $H_2$ by reduction. This reductive desulphurisation may be carried out in

the presence of a suitable conventional hydrogenation catalyst, such as Raney nickel, under conventional conditions for such reactions. For example a solution of the chosen compound of the formula (I) wherein X is S in an organic solvent may be added to a refluxing suspension of the catalyst in a similar solvent.

Also for example if desired compounds wherein Y is -C≡C- may be reduced to compounds wherein Y is cis--CH=CH- in known manner. Suitably this reaction is carried out using catalytic hydrogenation, such as Lindlar catalysis. When Y is -CH=CH-, it may be reduced to $-CH_2-CH_2-$ in known manner, suitably using catalytic hydrogenation such as transition metal catalysis.

Similarly protected $R_3$ hydroxy moieties may be deprotected in conventional manner. For example when $R_3$ is a benzyloxy group, the benzyl group may readily be removed by hydrogenolysis. Thus it may be seen that 'protected hydroxy' compounds of the formula (I) are useful intermediates in the preparation of the corresponding 'free hydroxy' compounds of the formula (I).

Also when a compound of the formula (I) contains (an)acidic hydrogen atom(s), salts thereof may be prepared in conventional manner for example by reacting the compound of the formula (I) with the required base. For salts of compounds wherein $R_5$ is hydrogen, the base should be a strong base such as for example sodium in an alcohol, such as ethanol, or the like.

It will be appreciated that when X is $H_2$, Y may conveniently be $-CH,CH_2-$.

Compounds of the formula (I) have particularly useful pharmacological activity. For example compounds of the formula (I) have anti-gastric secretion activity; anti-ulcer activity; activity on smooth muscle, such as vascular activity, eg anti-hypertensive activity, effects on the respiratory tract, eg bronchodilation activity, anti-fertility activity and gastrointestinal smooth muscle activity; platelet aggregation inhibition activity and/or cardiac activity, eg anti-arrhythmic activity and/or anti-hypertensive activity.

Compounds of the formula (I) may accordingly be used in the treatment of the corresponding disorders in humans and animals.

The compounds of the formula (I) are especially useful bronchodilation agents.

In general it may be said that compounds of the formula (I) have a range of pharmacological activities similar to those shown by the natural prostaglandins, but that their activity profiles tend to be rather more selective, so that each compound tends to have a major activity readily ascertained by routine pharmacological tests.

The invention therefore also provides a pharmaceutical composition comprising a compound of the formula (I) and a pharmaceutically acceptable carrier.

The compounds of the formula (I) also have good stability.

In order to utilise the selectivity of activity found with compounds of the formula (I), normally a given compound will be used in the treatment of the disorder corresponding to the compound's major activity (that is, the disorder for which the compound has the lowest active dose) and will accordingly be formulated into the corresponding pharmaceutical composition, and administered in a manner conventional for treatment of that disorder. It may also of course be possible with compounds having one or more further pronounced activities to formulate and use the compound for those further activities as well as for the major activity, provided that there is no undesirable pharmacological interaction between the different activities, or that separation of the different activities can be obtained by a difference in the formulation or in the mode of administration.

The composition may be in the form of tablets, capsules, powders, granules, lozenges or liquid preparations, such as oral or sterile parenteral solutions or suspension.

Tablets and capsules for oral administration may be in unit dose presentation form, and may contain conventional excipients such as binding agents, fillers, tabletting lubricants, disintegrants, and acceptable wetting agents and the like. The tablets may be coated according to methods well known in normal pharmaceutical practice. Oral liquid preparations may be in the form of, for example, aqueous or oily suspensions, emulsions, syrups or elixirs, or may be presented as a dry product

for reconstitution with water or other suitable vehicle before use. Such liquid preparations may contain conventional additives such as suspending agents, emulsifying agents, non-aqueous vehicles (which may include edible oils), preservatives, and if desired conventional flavouring or colouring agents, and the like.

For parenteral administration, fluid unit dosage forms are prepared utilising the compound of the formula (I) and a sterile vehicle. The compound, depending on the vehicle and concentration used, can be either suspended or dissolved in the vehicle. In preparing solutions the compound can be dissolved for injection and filter sterilised before filling into a suitable vial or ampoule and sealing. Advantageously, adjuvants such as a local anaesthetic, preservatives and buffering agents can be dissolved in the vehicle. Parenteral suspensions are prepared in substantially the same manner except that the compound is suspended in the vehicle instead of being dissolved and sterilisation cannot be accomplished by filtration. The compound can be sterilised by exposure to ethylene oxide before suspending in the sterile vehicle. Advantageously a surfactant or wetting agent is included in the composition to facilitate uniform distribution of the compound.

When appropriate, the compositions of this invention may be presented as an aerosol for oral administration, or as a microfine powder for insufflation.

As is common practice, the compositions will usually be accompanied by written or printed directions for use in the medical treatment concerned.

It will of course be ralised that the precise dosage used in the treatment of any of the hereinbefore described disorders will depend on the actual compound of the formula (I) used, and also on other factors such

as the seriousness of the disorder being treated.

The invention also provides a method of treatment and/or prophylaxis of disorders in human being or animals which comprises the administration to the sufferer of an effective amount of a compound of the formula (I). Normally however the compounds will be used in the therapy of human disorders.

The following Examples illustrate the preparation of compounds of the formula (I) and the following descriptions illustrate the preparation of intermediates thereto.

Example 1

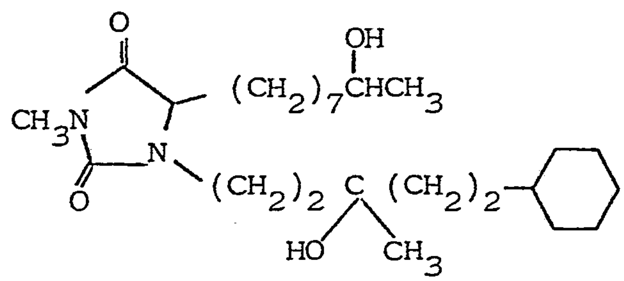

Compound 1

Sodium borohydride (770 mg; 1:1 eq) was added in portions to a solution of 1-(3'-hydroxy-3'-methyl-5'-cyclohexyl-n-pentyl)-3-methyl-5-(8"-oxo-nonyl) hydantoin in ethanol (10 ml). The mixture was stirred at room temperature for three hours. The resulting solution was partitioned between ether and very dilute hydrochloric acid. The ether solution was washed with 5% sodium bicarbonate and with brine then was dried and evaporated *in vacuo* to give a pale yellow oil (750 mg). The oil was chromatographed on silica gel (25 g) using chloroform and 1% methanol chloroform as eluants, to give

1-(3'-hydroxy-3'-methyl-5'-cyclohexyl-n-pentyl)-3-methyl-5-(8"-hydroxy-n-nonyl) hydantoin as a clear oil (659 mg).

Compounds 2 to 5 shown in Table 1 were prepared in a similar manner.

Table 1

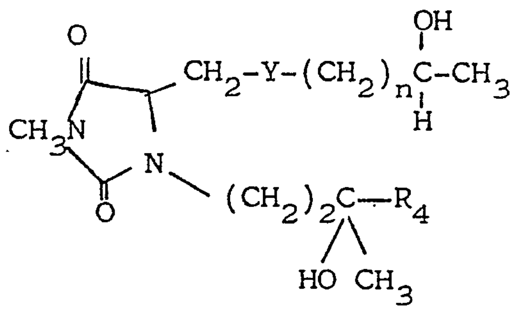

| Compound | Y | n | $R_4$ |
|---|---|---|---|
| 1 | $-CH_2-CH_2-$ | 4 | $(CH_2)_2$ 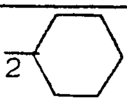 |
| 2 | $-CH_2-CH_2-$ | 4 | $C_6H_{13}$ |
| 3 | $-CH\equiv CH-$ | 4 | $C_6H_{13}$ |
| 4 | $-CH_2-CH_2-$ | 5 | $C_6H_{13}$ |
| 5 | $-CH_2-CH_2-$ | 4 | 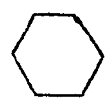 |

Analytical Data for Compounds in Table 1

Compound 1

I.R. ($cm^{-1}$) film: 3430 [OH]; 1760, 1710 $[N-\overset{O}{\overset{\|}{C}}-N-\overset{O}{\overset{\|}{C}}]$.

N.M.R. (τ) $CDCl_3$: 8.2, bs, 2H, [OH];
7.0, s, 3H, $[NCH_3]$;
6.5, centre of brm, 2H, $[NCH_2]$;
6.3, m, 1H, [CHOH];
6.0, m, 1H, [NCH].

Mass Spectrum: $C_{25}H_{46}N_2O_4$ ($m^*$) requires : 438.3456
found : 438.3436.

Compound 2

I.R. ($cm^{-1}$) film: 3430 [OH]; 1765, 1700 $[N-\overset{O}{\overset{\|}{C}}-N-\overset{O}{\overset{\|}{C}}]$.

N.M.R. (τ): 7.55, s, 2H, [OH];
7.0, s, 3H, $[NCH_3]$;
6.5, centre of brm, 2H, $[NCH_2]$;
6.25, m, 1H, [CHOH];
5.95, m, 1H, [NCH].

Mass Spectrum: $C_{23}H_{44}N_2O_4$ ($m^*$) requires: 412.3299
found : 412.3308.

Compound 3

I.R. ($cm^{-1}$) film: 3430 [OH]; 1760, 1700 $[N-\overset{O}{\overset{\|}{C}}-N-\overset{O}{\overset{\|}{C}}]$.

N.M.R. ($\tau$) $CDCl_3$: 7.95, bs, 2H, [OH];

7.95, m, 2H, $[CH{=}CH\underline{CH_2}]$;

7.45, m, 2H, $[\underline{CH_2}CH{=}CH]$;

7.0, s, 3H, $[NCH_3]$;

6.5, centre of brm, 2H, $[NCH_2]$;

6.25, m, 1H, $[C\underline{H}OH]$;

6.0, m, 1H, [NCH];

4.65, m, 2H, [CH=CH].

Mass Spectrum: $C_{23}H_{42}N_2O_4$ ($m^*$) requires: 410.3142

found : 410.3118

Compound 4

I.R. ($cm^{-1}$; film): 3430 [OH]; 1765, 1700 $[N-\overset{O}{\overset{\|}{C}}-N-\overset{O}{\overset{\|}{C}}]$.

N.M.R. ($\tau$; $CDCl_3$): 7.35, s, 2H, [OH];

7.0, s, 3H, $[NCH_3]$;

6.5, centre of brm, 2H, $[NCH_2]$;

6.3, m, 1H, $[C\underline{H}OH]$;

6.0, m, 1H, [NCH].

Mass Spectrum: $C_{24}H_{46}N_2O_4$ ($M^*$) requires: 426.3456

found : 426.3480

<u>Compound 5</u>

I.R. ($cm^{-1}$; film): 3430 [OH]; 1760, 1700 $[N-\overset{O}{\overset{\|}{C}}-N-\overset{O}{\overset{\|}{C}}]$

Mass Spectrum: $C_{23}H_{40}N_{2}O_{3}$ ($M^{*}$) requires: 392.3037

found : 392.3020

Example 2

Description 1

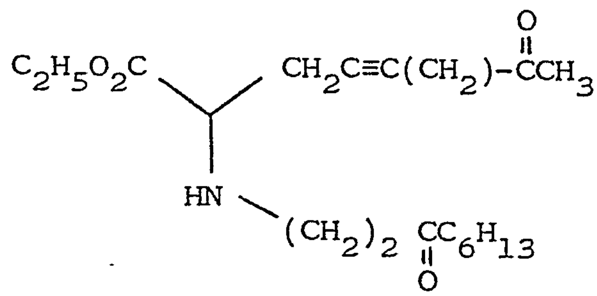

Non-1-ene-3-one (1.22 g) was added dropwise to stirred, ice-cold, ethyl 2-amino-10-oxo-undec-4-ynoate (3.1 g) and the mixture was stirred at room temperature for three hours. The resulting oil was chromatographed on silica gel (65 g) using ethyl acetate/hexane (2/3) as eluant to give ethyl 2-(3'-oxo-nonyl) amino-10-oxo-undec-4-ynoate (1.9 g) as a yellow oil.

I.R. ($cm^{-1}$; film): 1730 ($CO_2C_2H_5$); 1710 ($>C=O$).

N.M.R. (τ; $CDCl_3$): 7.52, s, 3H, ($COCH_3$);

7.6 to 7.0, m, 12H, [($CH_2\overset{O}{\overset{\|}{C}}$ x 3); $NCH_2$; $CH_2C\equiv CCH_2$];

6.65, t, 1H, (NCH);

5.85, q, 2H, ($CO_2\underline{CH_2}CH_3$).

Description 2

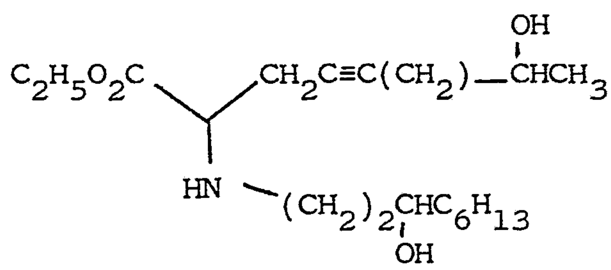

Sodium borohydride (0.42g; 2.2 mol. equiv) was added in portions to a solution of ethyl 2-(3'-oxo-nonyl) amino-10-oxo-undec-4-ynoate (1.9 g) in dry ethanol (25 ml) at $0^{o}C$. The mixture was stirred at room temperature for three hours then was partitioned between very dilute hydrochloric acid and ether. The ether solution was washed with 5% sodium bicarbonate solution and with brine and dried and evaporated *in vacuo* to give a pale yellow oil (1.9 g). The oil was chromatographed on silica gel (27.5 g) using 50% ethyl acetate/ hexane as eluant to give ethyl 2-(3'-hydroxy-nonyl)amino -10-hydroxy-4-ynoate (700 mg) as a pale yellow oil.

Example 2

Compound 6

1-(3'-Hydroxy-nonyl)-3-methyl-5-(8"-hydroxy-non-2-ynyl)-2-thiohydantoin

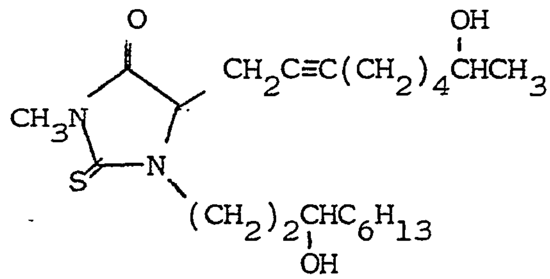

Methyl isothiocyanate (133 mg) in dry toluene (10 ml) was added to a stirred solution of ethyl 2-(3'-hydroxy-nonyl)amino-10-hydroxy-4-ynoate (700 mg) in dry toluene (50 ml) at $0^{o}C$. The solution was stirred at $0^{o}C$ for ½ hour, at room temperature for 2 hours, then at reflux for 2 hours. The cooled solution was evaporated *in vacuo* and the resulting oil was chromatographed on silica gel (16 g) using ethylacetate/pentane (2/3) as eluant to give 1-(3'-hydroxy-nonyl)-3-methyl-5-(8"-hydroxy-non-2-ynyl)-2-thiohydantoin (566 mg) as a pale yellow gum.

I.R. ($cm^{-1}$; film): 3400 (OH); 1740 (>NC(S)NC(O)- ).

N.M.R. (τ; $CDCl_3$):
7.9, m, 2H, ($C\equiv C\ CH_2$);
7.15, m, 2H, ($CH_2\ C\equiv C$);
6.75, s, 3H, ($NCH_3$);
6.7 to 5.9, m, 6H, [(CHOH x 2); $NCH_2$];
5.9, m, 1H, (NCH).

Mass Spectrum: $C_{22}H_{38}N_2O_3S$ ($M^+$) requires: 410.2603
found : 410.2601

## Pharmacological Data Section

### Bronchodilator Activity

The compounds were examined for their ability to inhibit 5-hydroxy tryptamine induced bronchoconstriction in the anaethetised, artifically respired guinea pig (Konzett-Rossler preparation). The compounds were administered intravenously.

| Compound | $ED_{50}$/ µg/kg i.v. |
|---|---|
| 2 | 5.5 |
| 3 | 3.6 |
| 5 | 24.0 |

### Toxicity

No toxic effects were observed during this test.

Claims

1. A compound of formula (I):

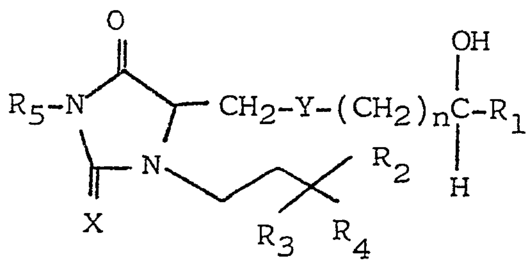

(IO

wherein:

X is O, S or $H_2$;

Y is $-CH_2-CH_2-$, $-CH=CH-$ or $-C\equiv C-$;

n is 1 to 5;

$R_1$ is $C_{1-4}$ alkyl;

$R_2$ is hydrogen or $C_{1-4}$ alkyl;

$R_3$ is a hydroxy or protected hydroxy;

$R_4$ is $C_{1-9}$ alkyl, $C_{3-8}$ cycloalkyl or $C_{3-8}$ cycloalkyl-$C_{1-6}$ alkyl;

$R_4$ is hydrogen or $C_{1-6}$ alkyl; or a salt thereof.

2. A compound according to claim 1 of formula (II):

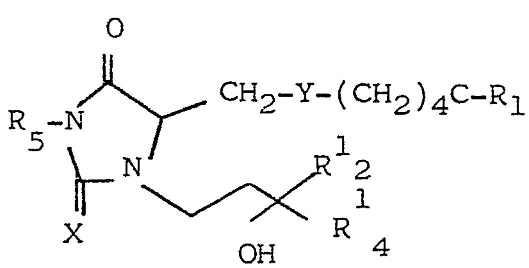

(II)

wherein:

X, Y, $R_1$ and $R_5$ are as defined in claim 1;

$R^1_2$ is hydrogen, methyl or ethyl; and

$R^1_4$ is $C_{1-9}$ alkyl; or a salt thereof.

3. A compound according to claim 2, wherein $R^1_4$ is straight chain pentyl, hexyl or heptyl.

4. 1-(3'-hydroxy-3'-methyl-n-nonyl)-3-methyl-5-(8"-hydroxy-n-nonyl)hydantoin or 1-(3'-hydroxy-3'-methyl-n-nonyl)-3-methyl-5-(cis-8"-hydroxy-n-hex-2"-enyl)hydantoin.

5. A compound according to claim 1 of the formula (III):

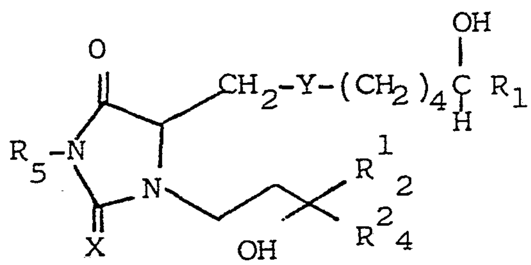

(III)

wherein:

X, Y, $R_1$ and $R_5$ are as defined in claim 1;

$R^1_2$ is hydrogen, methyl or ethyl;

$R^2_4$ is a group of formula (IV):

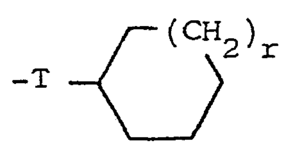

wherein T is a bond, or a $C_{1-6}$ alkylene group which may be straight chain or branched by one or two methyl groups at the same or different carbon atoms; and

r is 0 to 3;

or a salt thereof.

6. A compound according to claim 5 wherein r is 1 and T is a bond.

7. 1-(3'-hydroxy-3'-cyclohexyl-n-butyl)-3-methyl-5-(8"-oxo-n-nonyl)hydantoin.

8. A pharmaceutical composition comprising a compound according to claim 1 together with a pharmaceutically acceptable carrier.

9. A pharmaceutical composition comprising the compound of claim 7 together with a pharmaceutically acceptable carrier.

10. A process for the preparation of a compound according to claim 1 characterised by either

i) the cyclisation of a compound of formula (V):

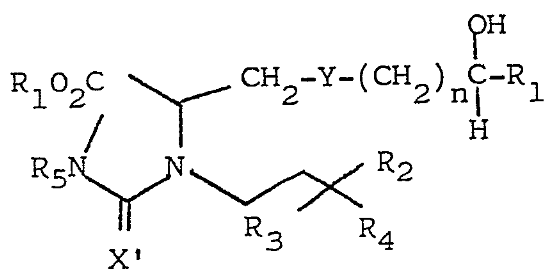

(V)

wherein the variable groups are as defined in claim 1 and X' is O or S; optionally formed <u>in situ</u> during the reaction of a compound of the formula (VI):

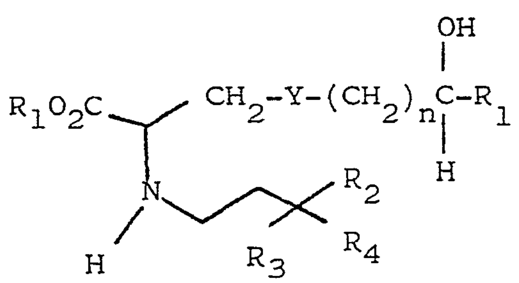

with either $R_5NCX'$ when $R_5$ is $C_{1-6}$ alkyl; or with $M^+\bar{C}NX$ wherein M+ is a metal ion in the presence of acid when $R_5$ is hydrogen; and thereafter if desired or necessary converting X, Y, $R_1$, $R_3$ and $R_5$ in the thus formed compound into other variables X, Y, $R_1$, $R_3$ and $R_5$

or

ii) for a compound of the formula (I) wherein X is O, reacting a compound of formula (X):

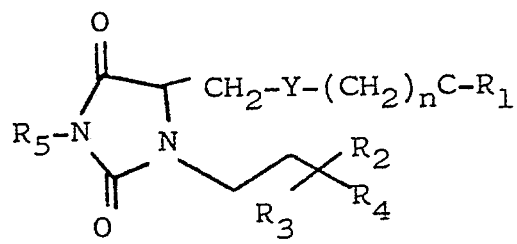

(X)

wherein the variables are as defined in claim 1 with a carbonyl-reducing agent, and thereafter if desired or necessary converting Y, $R_1$, $R_3$ and $R_5$ in the thus formed compound into other variables Y, $R_1$, $R_3$ and $R_5$.

Claims

1. A process for the preparation of a compound of formula (I):

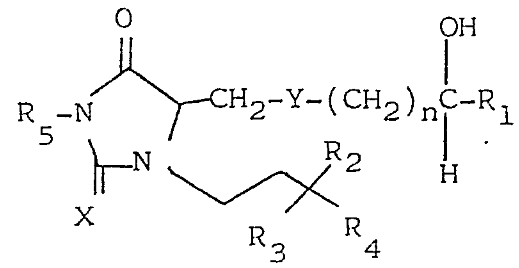

(I)

wherein:

X is O, S or $H_2$;

Y is $-CH_2-CH_2-$, $-CH=CH-$ or $-C\equiv C-$;

n is 1 to 5;

$R_1$ is $C_{1-4}$ alkyl;

$R_2$ is hydrogen or $C_{1-4}$ alkyl;

$R_3$ is a hydroxy or protected hydroxy;

$R_4$ is $C_{1-9}$alkyl, $C_{3-8}$ cycloalkyl or $C_{3-8}$ cycloalkyl-$C_{1-6}$ alkyl; and

$R_5$ is hydrogen or $C_{1-6}$alkyl; and slats thereof characterised by either

i) the cyclisation of a compound of formula (V):

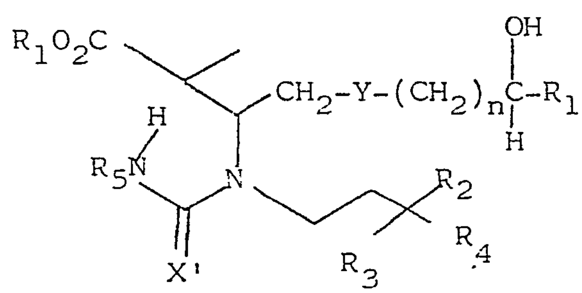

(V)

wherein the variable groups are as hereinbefore defined and X' is O or S; optionally formed <u>in situ</u> during the reaction of a compound of the formula (VI):

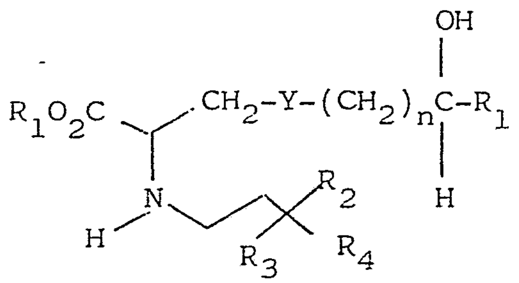

(VI)

with either $R_5NCX'$ when $R_5$ is $C_{1-6}$ alkyl; or with $M^+\bar{C}NX$ wherein $M^+$ is a metal ion in the presence of acid when $R_5$ is hydrogen

or

ii) for a compound of the formula (I) wherein X is O, reacting a compound of formula (X):

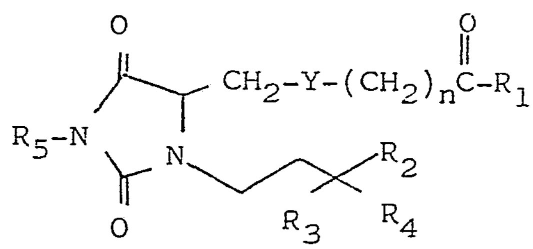

(X)

wherein the variables are as hereinbefore defined with a carbonyl reducing agent and thereafter if desired or necessary converting Y, $R_1$, $R_3$ and $R_5$ in the thus formed compound into other variables Y, $R_1$, $R_3$ and $R_5$.

2. A process according to claim 1 characterised in that
$R_2$ is hydrogen, methyl or ethyl;
$R_3$ is hydroxy; and
$R_4$ is $C_{1-9}$ alkyl

3. A process according to claim 2, characterised in that X is O and $R_4$ is straight chain pentyl, hexyl or heptyl.

4. A process according to claim 3 for the preparation of

1-(3'-hydroxy-3'-methyl-*n*-nonyl)-3-methyl-5-(8"-hydroxy-*n*-nonyl)hydantoin or 1-(3'-hydroxy-3'-methyl-*n*-nonyl)-3-methyl-5-(8"-hydroxy-*n*-*cis*-2"-enyl)hydantoin.

5. A process according to claim 1 characterised in that

$R_2$ is hydrogen, methyl or ethyl;

$R_4$ is a group of formula (IV):

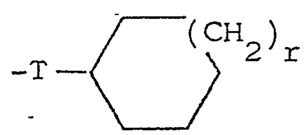

(IV)

wherein T is a bond, or a $C_{1-4}$ alkylene group which may be straight chain or branched by one or two methyl groups at the same or different carbon atoms; and

r is 0 to 3;

6. A process according to claim 5 characterised r is 1 and T is a bond.

7. A process according to claim 1 for the preparation of 1-(3'-hydroxy-3'-cyclohexyl-*n*-butyl)-3-methyl-5-(8"-oxo-*n*-nonyl)hydantoin.

0023083

European Patent Office

# EUROPEAN SEARCH REPORT

Application number

EP 80302012.2

| Category | Citation of document with indication, where appropriate, of relevant passages | Relevant to claim | CLASSIFICATION OF THE APPLICATION (Int. Cl.) |
|---|---|---|---|
| | EP - A1 - 0 001 238 (WELLCOME) (04-04-1979)<br>* Pages 1-7,18 *<br>-- | 1,8,10, AT 1 | C 07 D 233/78<br>C 07 D 233/86<br>C 07 D 233/32<br>A 61 K 31/415 |
| | EP - A2 - 0 002 259 (WELLCOME) (13-06-1979)<br>* Pages 1-6,10 *<br>-- | 1,8,10, AT 1 | |
| P | DE - A1 - 2 922 070 (WELLCOME) (06-12-1979)<br>* Claims; pages 25,26 *<br>& BE-A4- 876 670 (30-11-1979)<br>& FR-A2-2 427 331 (28-12-1979)<br>& NL-A -7 904 311 (04-12-1979)<br>& SE-A -7 904 700 (01-12-1979)<br>---- | 1,8,10, AT 1 | |

TECHNICAL FIELDS SEARCHED (Int.Cl.)

C 07 D 233/00
A 61 K 31/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 05-09-1980 | PETROUSEK |

EPO Form 1503.1 06.78